# EUROPÄISCHE PATENTSCHRIFT

(11) **EP 1 237 478 B1**
(45) Veröffentlichungstag und Bekanntmachung des Hinweises auf die Patenterteilung: **12.11.2003**
(21) Anmeldenummer: 00929421.6
(22) Anmeldetag: 25.04.2000
(51) Int. Cl.: A61B 5/083

(54) **VERFAHREN UND VORRICHTUNG ZUR ATEMZUGSAUFGELÖSTEN BESTIMMUNG DES PARTIALDRUCKS EINER GASKOMPONENTE IN DER AUSATEMLUFT EINES PATIENTEN**
METHOD AND DEVICE FOR DETERMINING THE PARTIAL PRESSURE OF A GAS COMPONENT IN THE EXPIRATION AIR OF A PATIENT, RESOLVED PER RESPIRATION
PROCEDE ET DISPOSITIF DE DETERMINATION, DECOMPOSEE PAR RESPIRATION, DE LA PRESSION PARTIELLE D'UN CONSTITUANT GAZEUX DANS L'AIR D'EXPIRATION D'UN PATIENT

(43) Veröffentlichungstag der Anmeldung: 11.09.2002
(73) Patentinhaber: EnviteC-Wismar GmbH, 23966 Wismar (DE)
(72) Erfinder: LINDNER, Bernd, D-23626 Ratekau (DE)
(74) Vertreter: Best, Michael, Dr.
(86) Internationale Anmeldenummer: EP0003689
(87) Internationale Veröffentlichungsnummer: WO01080735

(56) Entgegenhaltungen:
- EP-A- 0 392 503
- WO-A-93/00040
- WO-A-99/39637
- DE-A- 4 001 803
- GB-A- 2 084 321
- US-A- 3 523 529
- US-A- 4 359 057
- US-A- 4 572 208

## Beschreibung

Die Erfindung betrifft ein Verfahren und eine Vorrichtung zur atemzugsaufgelösten Bestimmung des Partialdrucks einer Gaskomponente in der Atemluft eines Patienten, genauer, ein Verfahren zur Bestimmung des CO₂-Gehalts in der Atemluft sowie eine Beatmungsvorrichtung, die so ausgestaltet ist, daß das Verfahren damit ausgeführt werden kann.

In der Medizin lassen sich aus dem Kohlendioxidgehalt in der Atemluft wichtige Schlußfolgerungen über den Zustand eines Patienten und mögliche gesundheitliche Störungen ziehen. Neben dem Absolutgehalt (oder dem Partialdruck) des Kohlendioxids in der Ausatemluft ist hierbei die CO₂-Kurvenform wesentlich (entsprechende Kurven, bei denen der CO₂-Gehalt (oder -Partialdruck) gegen die Zeit aufgetragen ist, bezeichnet man als Kapnogramm), insbesondere aber auch ob der Maximalwert des Kohlendioxidgehalts in der Ausatemluft im Laufe der Atemzyklen ansteigt, abfällt oder konstant bleibt und mit welcher Geschwindigkeit er ansteigt bzw. abfällt. Diese Informationen sind insbesondere wichtig, wenn ein Patient intubiert und künstlich beatmet wird, beispielsweise unter Vollnarkose, aber auch in der Notfallmedizin und bei Spontanatmung.

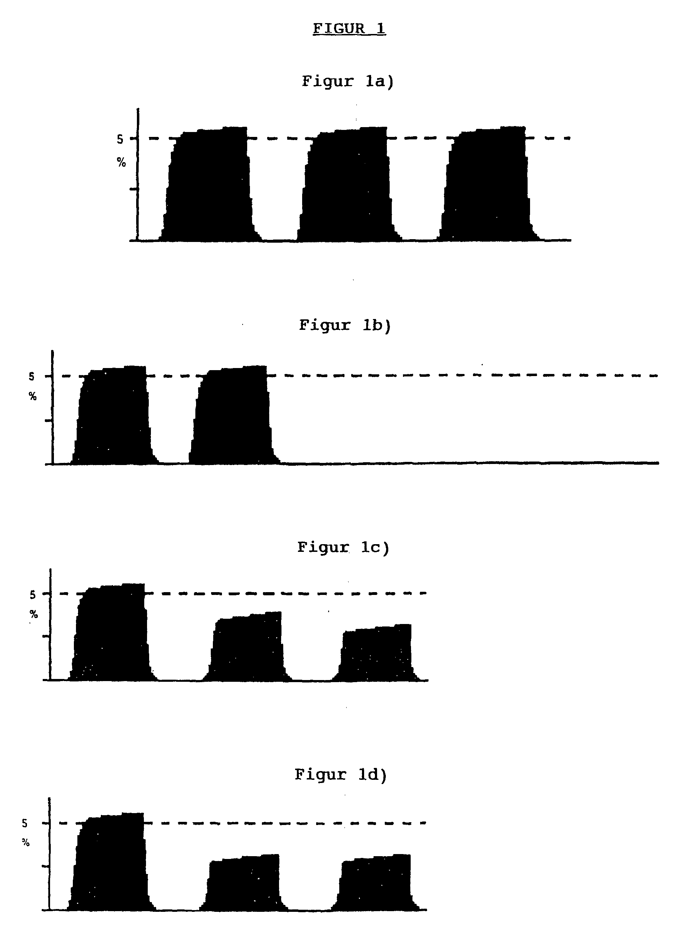

In Figur 1 sind einige Beispiele typischer Kapnogramme dargestellt. Dabei zeigt Figur 1a) das Kapnogramm eines gesunden Patienten unter kontrollierter Beatmung. Der maximale CO₂-Gehalt in der Ausatemluft beträgt circa 5%. Figur 1b) zeigt das Kapnogramm eines Patienten, bei dem die normale CO₂-Kurve von einem Atemzug zum nächsten auf 0 fällt. Dies kann beispielsweise durch eine Diskonnektion des Beatmungsgeräts vom Patienten verursacht sein, oder es kann eine vollständige Luftwegsobstruktion aufgetreten sein, die z.B. durch einen völlig blockierten endotrachialen Tubus hervorgerufen wird. Figur 1c) zeigt ein rapides ständiges Abfallen des CO₂-Gehalts in der Ausatemluft und kann ein Hinweis sein für eine signifikante pulmonale Luftembolie, einen Herzstillstand oder eine schwere Hypotension. Bei Figur 1d) fällt der Kohlendioxidgehalt in der Atemluft plötzlich auf einen niedrigeren Stand aber nicht auf 0 und bleibt dort konstant. Dies wird beispielsweise durch eine Verlagerung des endotrachialen Tubus in einen Bronchus z.B. während der Änderung der Lage des Patienten oder durch eine plötzliche partielle Luftwegsobstruktion verursacht. Ein Kapnogramm gibt auch Hinweise auf einsetzende Hyperventilation, sinkendes Herzminutenvolumen oder Rückgang der pulmonalen Perfusion, Beginn einer Hypoventilation, eine zunehmende Energieumsatzrate in Folge von Schmerzen oder Fieber, eine versehentliche Intubierung des Magens statt der Lunge, maligne Hyperthermie, unzureichende Muskelrelaxation und nicht ausreichende Tiefe der Narkose und andere ernste oder lebensbedrohende Zustände des Patienten.

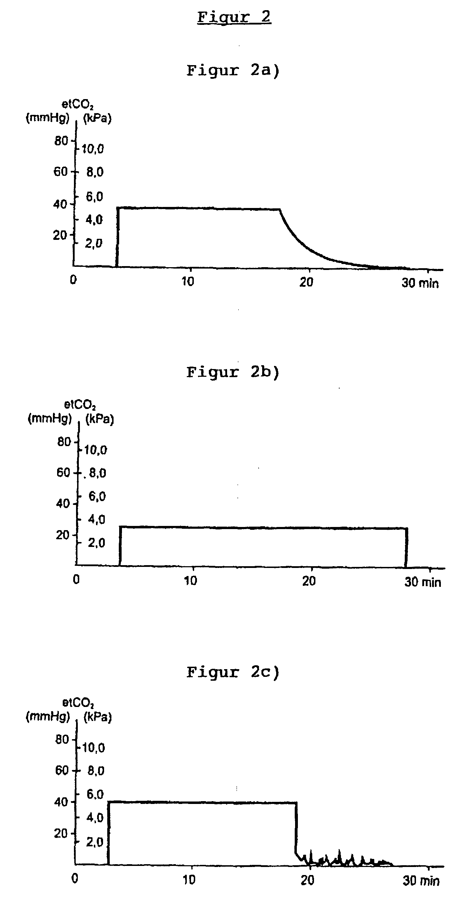

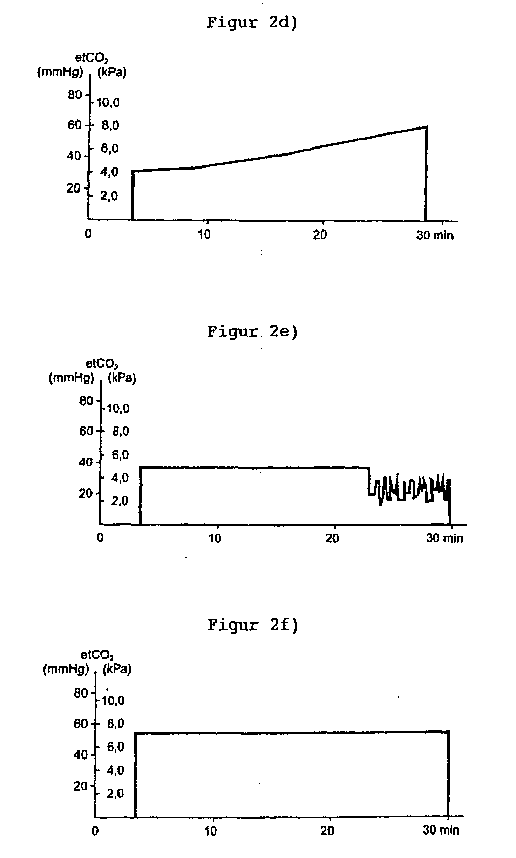

Auch wenn kein vollständiges Kapnogramm zur Verfügung steht, kann aus der Entwicklung des maximalen Kohlendioxidgehalts in der Ausatemluft über mehrere Atemzyklen hinweg wertvolle Informationen über möglicherweise ernste oder lebensbedrohliche Zustände eines Patienten erhalten werden. Entsprechende sogenannte Trendkurven sind in Figur 2 gezeigt. In Figur 2a) ist der Patient zunächst stabil, und der Maximalgehalt des Kohlendioxids in der Ausatemluft beträgt circa 5%. Der Maximalgehalt des Kohlendioxids in der Ausatemluft fällt jedoch plötzlich rapide ab. Mögliche Ursachen hierfür sind ein cardiopulmonaler Bypass, Herzstillstand, pulmonale Embolie, hoher Blutverlust oder ein schlagartiger Abfall des Blutdrucks. Figur 2b) zeigt einen konstant erniedrigten maximalen Kohlendioxidgehalt in der Ausatemluft, der bei knapp 4% liegt. Mögliche Ursachen hierfür sind Hyperventilation durch zu hohes Minutenvolumen oder niedrige Körpertemperatur nach Schock. Figur 2c) zeigt den plötzlichen Abfall des Maximalwerts des Kohlendioxidgehalts in der Ausatemluft auf etwa 0. Hierfür sind mögliche Ursachen akzidentelle Extubation, vollständiger Atemwegsverschluß, Diskonnektion oder Ösophagusintubation. Bei einer Ösophagusintubation erfolgt der Abfall auf 0 bereits nach ein bis zwei Atemzyklen. Figur 2d) zeigt einen graduellen Anstieg des Maximalwerts der CO₂-Konzentration in der Ausatemluft, möglicherweise hervorgerufen durch die Erhöhung des Stoffwechsels und der Körpertemperatur, eine beginnende Hypoventilation oder durch die Abnahme der effektiven Alveolarventilation. Figur 2e) zeigt die Trendkurve bei einem plötzlichen Abfall des Maximalwerts des Kohlendioxidgehalts in der Ausatemluft, beispielsweise durch eine Leckage im Schlauchsystem, einen partiellen Atemwegsverschluß oder durch einen Tubus in hypopharynx hervorgerufen. Figur 2f) zeigt einen konstant erhöhten Maximalwert des Kohlendioxids in der Ausatemluft, für den mögliche Ursachen eine Atemdepression durch Medikamente, metabolische Alkalose (respiratorische Kompensation) oder eine unzureichende Minutenventilation ist.

Zur CO₂-Bestimmung in der Atemluft in der Medizin kann auf "Annals of Emergency Medicine" 1989, 1287/53 bis 1290/56, "Annals of Emergency Medicine" 1989, 166/1375, "Prehospital and Disaster Medicine" Vol. 4, # 1, 1989, Seite 74 und "JAMA" 1987, Vol. 257, Nr. 4, 512 bis 515" verwiesen werden.

Geräte zur Bestimmung des Kohlendioxidgehalts in der Atemluft sind bekannt und werden in der Medizin vielfältig eingesetzt. Diese Geräte müssen schnell auf Änderungen des CO₂-Gehalts in der Atemluft ansprechen, und in der Regel werden hierfür Geräte auf Grundlage einer Infrarotabsorptionsspektroskopie verwendet. Vorrichtungen, die derartige Kohlendioxidsensoren einsetzen, sind beispielsweise in der EP-A 392 503, der DE-A 35 33 557 und der DE-A 31 37 258 beschrieben.

Es sind auch Geräte bekannt, bei denen der CO₂-Gehalt als Farbumschlag eines Indikatorsystems angezeigt wird. Derartige Vorrichtungen sind beispielsweise in der US-A 4,728,499 beschrieben und kommerziell erhältlich.

Die CO₂-Detektoren auf Grundlage von IR-Absorptionsspektroskopie haben den Vorteil, daß sie eine sehr schnelle Ansprechzeit haben und den CO₂-Gehalt in der Atemluft mit sehr hoher Auflösung wiedergeben. Derartige Geräte sind aber ausgesprochen teuer, und ihr Einsatz, insbesondere in der Notfallmedizin, beispielsweise in Krankenwagen, ist in der Regel aus Kostengründen nicht möglich. Die CO₂-Detektoren auf Grundlage einer Farbindikatorreaktion sind zwar preiswerter, zeigen aber keine Trendinformation und sind, da Farbvergleiche notwendig sind, verhältnismäßig ungenau und schwierig abzulesen. Mit derartigen CO2-Detektoren auf Grundlage einer Farbindikatorreaktion ist es auch nicht möglich, Kapnogramme zu erstellen.

Es besteht in der Medizin ein erheblicher Bedarf nach einer preiswerten Vorrichtung, mit der der Kohlendioxidgehalt in der Atemluft bestimmt werden kann. Das Verfahren soll atemzugsaufgelöst arbeiten, das heißt bei jedem Atemzyklus soll in der Einatemluft und in der Ausatemluft der Kohlendioxidgehalt (das heißt der Kohlendioxidpartialdruck) bestimmt werden. Das Verfahren soll in der Lage sein, eine Trendkurve, zumindest aber eine Trendinformation anzuzeigen, das heißt ob sich der maximale Kohlendioxidgehalt in der Ausatemluft bei aufeinanderfolgenden Atemzyklen verringert, vergrößert oder gleich bleibt und mit welcher Geschwindigkeit dies geschieht. Es besteht ebenfalls Bedarf nach einer Vorrichtung zur Durchführung eines derartigen Verfahrens.

US 3,523,529 offenbart einen polarographischen Sauerstoffsensor, welcher dazu verwendet wird, ein elektrisches Signal zu erzeugen, das proportional zu der Änderung des prozentualen Sauerstoffgehalts der eingeatmeten Luft zu dem der ausgeatmeten Luft ist.

Aus der DE-A 40 01 803 ist ein Verfahren zur Bestimmung der Kohlendioxidproduktion im Atemgas bekannt. Hierbei soll auf ein Kohlendioxidkonzentrationsmeßgerät verzichtet werden. Bei dem Verfahren wird über einen seriell ablaufenden Meßzyklus zunächst mit zwei Sauerstoffsensoren der Sauerstoffverbrauch und die Sauerstoffkonzentrationswerte mit CO₂-Absorber und ohne CO₂-Absorber gemessen, und aus diesen Werten und der Sauerstoffkonzentration im Einatemzweig wird dann in der Steuereinheit die Kohlendioxidproduktion errechnet. Ein atemzugsaufgelöstes Messen des Kohlendioxidgehalts bzw. des Kohlendioxidpartialdrucks in der Ausatemluft ist mit der in der DE-A 40 01 803 beschriebenen Vorrichtung nicht möglich, und ein entsprechendes Verfahren ist nicht Gegenstand dieser Druckschrift. Die in der DE-A 40 01 803 beschriebene Bestimmung der Kohlendioxidproduktion im Atemgas darf nicht mit dem Gegenstand der vorliegenden Erfindung verwechselt werden, bei der es nicht um die Kohlendioxidproduktion geht, sondern um den Kohlendioxidgehalt in der Ausatemluft, der atemzugsaufgelöst bestimmt werden muß.

Aufgabe der Erfindung ist es, ein Verfahren und eine Vorrichtung zur Bestimmung des Kohlendioxidgehalts in der Atemluft zur Verfügung zu stellen, das die Probleme des Standes der Technik nicht aufweist und das insbesondere derart preiswert ist, daß es auch in Krankenwagen und Notarztwagen ohne weiteres eingesetzt werden kann und trotzdem zuverlässige Informationen über den Absolutwert und vor allem auch über den Trend des Kohlendioxidgehalts in der Atemluft über mehrere Atemzyklen liefert.

Diese Aufgabe wird durch den Gegenstand der Patentansprüche gelöst.

Das erfindungsgemäße Verfahren beruht darauf, daß man mit einem schnellen Sauerstoffsensor den Sauerstoffpartialdruck beim Atmen bestimmt. Aus dem Sauerstoffpartialdruck kann dann auf den Kohlendioxidgehalt in der Atemluft geschlossen werden. Beträgt beispielsweise der Sauerstoffpartialdruck in der Einatemluft 21 kPa und fällt der Sauerstoffpartialdruck in der Ausatemluft bis auf 16 kPa, entspricht in erster Näherung die Differenz im Sauerstoffpartialdruck von 5 kPa dem Maximalwert des Kohlendioxidpartialdrucks in der Ausatemluft. Bei dem erfindungsgemäßen Verfahren wird der Absolutwert der Differenz zwischen dem momentanen Sauerstoffpartialdruck in der Ausatemluft und dem Sauerstoffpartialdruck in der Einatemluft als Kohlendioxidgehalt in der Ausatemluft angegeben, wobei bei jedem Atemzyklus der Minimalwert des Sauerstoffpartialdrucks in der Ausatemluft bestimmt wird und der Absolutwert der Differenz des Minimalwerts des Sauerstoffpartialdrucks in der Ausatemluft und des Sauerstoffpartialdrucks in der Einatemluft als Maximalwert des Kohlendioxidgehalts in der Ausatemluft angezeigt wird. Durch kontinuierliche Messung des Sauerstoffpartialdrucks in der Atemluft kann man Kurven erhalten, die zu denen invers sind, wie sie beispielsweise in Figur 1 gezeigt sind, und nach einfacher Umrechnung können Kapnogramme erhalten und angezeigt werden.

In einer bevorzugten einfacheren und preiswerteren Ausführungsform, die insbesondere für den Einsatz in Krankenwagen und Notarztwagen gedacht ist, erfolgt keine Aufnahme und Darstellung des gesamten Kapnogramms, sondern es wird nur der Maximalwert des Kohlendioxidgehalts in der Ausatemluft atemzugsaufgelöst bestimmt und angezeigt. Darüberhinaus wird der Maximalwert des Kohlendioxidgehalts in der Ausatemluft bei jedem Atemzyklus mit den vorhergehenden Atemzyklen verglichen, und es wird eine Trendinformation angezeigt, das heißt ob der Maximalwert des Kohlendioxidgehalts in der Ausatemluft von Atemzyklus zu Atemzyklus steigt, fällt oder konstant bleibt. In Abhängigkeit dieser Information kann das Bedienpersonal dann gegebenenfalls geeignete Maßnahmen einleiten oder eingehende Untersuchungen veranlassen.

Im Prinzip kann jede bekannte Beatmungsvorrichtung so angepaßt werden, daß mit ihr das erfindungsgemäße Verfahren durchgeführt werden kann. Hierzu wird an einer bekannten Beatmungsvorrichtung ein Adapter angebracht, über den ein Sauerstoffsensor angeschlossen wird. Da erfindungsgemäß ausschließlich der Sauerstoffgehalt (Sauerstoffpartialdruck) bzw. der Kohlendioxidgehalt (Kohlendioxidpartialdruck) in der Atemluft bestimmt wird und nicht die Kohlendioxidproduktion oder der Sauerstoffverbrauch, ist zur Durchführung des erfindungsgemäßen Verfahrens ausschließlich ein Sauerstoffsensor notwendig, der an der Beatmungsvorrichtung angeschlossen ist. Ein komplizierter Meßaufbau und Abgleichvorrichtungen sowie die Verwendung mehrerer Sauerstoffsensoren, Mischkammern oder CO₂-Absorbern, wie sie bei der Bestimmung der Kohlendioxidproduktion erforderlich sind, wird erfindungsgemäß nicht benötigt und ist in den erfindungsgemäßen Vorrichtungen nicht vorhanden.

Für das erfindungsgemäße Verfahren bzw. die erfindungsgemäße Vorrichtung sollte ein möglichst schneller Sauerstoffsensor verwendet werden. Bevorzugt sind Sauerstoffsensoren mit einer Ansprechzeit von weniger als 500 Millisekunden, stärker bevorzugt mit einer Ansprechzeit von weniger als 200 Millisekunden. Besonders bevorzugt sind Sauerstoffsensoren mit einer Ansprechzeit von etwa 100 Millisekunden oder weniger. Je schneller die Ansprechzeit des Sauerstoffsensors ist, mit umso besserer Auflösung kann das Kapnogramm aufgezeichnet werden und umso mehr und genauere Informationen stehen dem Bedienpersonal zur Verfügung. Je schneller die Ansprechzeit des Sauerstoffsensors ist, desto genauer kann auch der Minimalwert des Sauerstoffpartialdrucks in der Ausatemluft, das heißt der Maximalwert des Kohlendioxidpartialdrucks in der Ausatemluft, bestimmt werden.

Schnelle Sauerstoffsensoren, die für das erfindungsgemäße Verfahren geeignet sind, sind bekannt und kommerziell erhältlich. Derartige Sauerstoffsensoren werden auch bereits in der Medizin eingesetzt, bislang aber noch nicht zur atemzugsaufgelösten Bestimmung des Kohlendioxidgehalts in der Atemluft. Hier können beispielsweise galvanische, paramagnetische oder optische Sauerstoffsensoren genannt werden. Auch Sauerstoffsensoren, die mit Laserdioden arbeiten, sind bekannt. Aus Kostengründen wird erfindungsgemäß allerdings ein schneller elektrochemischer Sauerstoffsenor bevorzugt, wie er beispielsweise von der Firma Teledyne Analytical Instruments und von der Anmelderin vertrieben wird.

Erfindungsgemäß kann der Sauerstoffsensor zwar im Prinzip an beliebiger Stelle der Beatmungsvorrichtung angebracht werden, die Messung sollte jedoch bevorzugt so körpernah wie möglich durchgeführt werden. In der Beatmungsvorrichtung ist dabei entweder bereits ein Adapterstück vorgesehen, an dem der Sauerstoffsensor angeschlossen werden kann, oder es wird an einen vorhandenen Anschluß zunächst noch ein Adapterstück für den Sauerstoffsensor angebracht. Eine Beatmungsvorrichtung kann sowohl einen Patiententubus enthalten, über den ein Patient künstlich beatmet wird, aber auch eine häufig im Notarzt- oder Krankenwagen eingesetzte Beatmungsmaske.

Das erfindungsgemäße Verfahren bzw. die erfindungsgemäße Vorrichtung sind sowohl bei einer künstlichen Beatmung des Patienten als auch bei Spontanatmung einsetzbar.

Der Sauerstoffsensor kann an eine Auswerte- und Anzeigevorrichtung angeschlossen werden, die das komplette Kapnogramm berechnet und anzeigt. Alternativ und erfindungsgemäß bevorzugt kann die Auswerte- und Anzeigevorrichtung auch nur den Minimalwert des Sauerstoffpartialdrucks bzw. Sauerstoffgehalts und damit den Maximalwert des CO₂-Gehalts bzw. des CO₂-Partialdrucks in der Ausatemluft bestimmen, eine Trendinformation berechnen und beides anzeigen. Die Trendinformation kann als Diagramm, wie z.B. in Abbildung 2 dargestellt, angegeben werden oder durch andere geeignete optische und/oder akustische Mittel, wie z.B. einen Pfeil, dessen Neigungswinkel von der waagrechten Ausrichtung ein Ansteigen oder Absinken des Maximalwerts des Kohlendioxidgehalts in der Ausatemluft anzeigt, gegebenenfalls gekoppelt mit einer akustischen Warnung, falls ein Ansteigen oder Absinken des Maximalwerts des Kohlendioxidgehalts in der Ausatemluft einen bestimmten Grenzwert überschreitet.

Zur Verbesserung des Rauschens ist es selbstverständlich auch möglich, daß die Auswertevorrichtung über einige Atemzüge mittelt und den gemittelten Wert anzeigt und/oder für die Berechnung der Trendinformation verwendet.

Auswerte- und Anzeigevorrichtungen, die die von Sauerstoffsensoren gelieferten elektrischen Signale entsprechend verarbeiten und darstellen können, sind im Prinzip bekannt und können von einem Fachmann auf übliche Art und Weise angepaßt und in die erfindungsgemäße Beatmungsvorrichtung integriert werden.

Man kann selbstverständlich durch Messen des Absolutdrucks das Meßsignal drucknormieren und damit das Gerät für den Einsatz in Flugzeugen oder Notfallhubschraubern vorsehen.

Im Rahmen dieser Anmeldung wird davon ausgegangen, daß die ausgeatmete Luft eine Temperatur von 37°C aufweist und eine relative Feuchtigkeit von 100%. Zur Erhöhung der Meßgenauigkeit kann die erfindungsgemäße Vorrichtung mit Temperatur- und/oder Feuchtigkeitssensoren ausgerüstet werden, die die Temperatur und/oder Feuchtigkeit der Einatemluft und/oder der Ausatemluft bestimmen. Der erfindungsgemäß gemessene Sauerstoffpartialdruck bzw. der daraus errechnete Kohlendioxidpartialdruck kann dann auf an sich bekannte Art und Weise zur Verringerung des Meßfehlers entsprechend den real gemessenen Temperatur und Feuchtigkeitswerten korrigiert werden.

Bei den vorstehenden Ausführungen wird davon ausgegangen, daß das dem Patienten zugeführte Luftgemisch im wesentlichen aus Sauerstoff und ansonsten inerten Gasen wie Stickstoff besteht.

Sollte der Patient mit einem Gasgemisch beatmet werden, das nicht inerte Bestandteile enthält, müssen die mit dem erfindungsgemäßen Verfahren berechneten Werte für den Kohlendioxidpartialdruck im Hinblick auf die nicht inerte Gaskomponente korrigiert werden. In der Praxis tritt dies insbesondere dann auf, wenn ein Patient mit einem Gasgemisch beatmet wird, das ein Anästhesiegas enthält. In diesem Fall muß der Partialdruck des Anästhesiegases in der Einatemluft und in der Ausatemluft bestimmt werden und bei der Bestimmung des Kohlendioxidpartialdrucks aus dem gemessenen Sauerstoffpartialdruck berücksichtigt werden. Bei der bevorzugten Ausführungsform des erfindungsgemäßen Verfahrens in der Notfallmedizin tritt diese Schwierigkeit nicht auf.

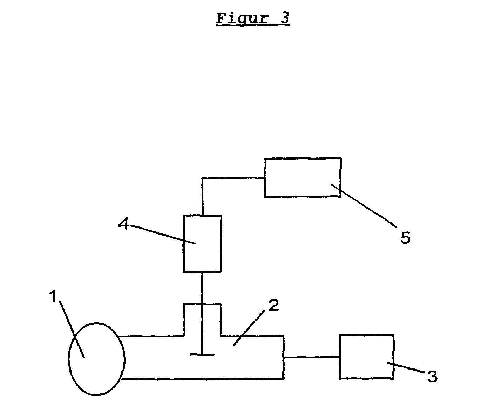

Die Erfindung wird im folgenden unter Bezugnahme auf Figur 3 näher beschrieben.

Figur 3 zeigt schematisch eine bevorzugte Ausführungsform einer Vorrichtung zur Durchführung des erfindungsgemäßen Verfahrens. In Figur 3 bedeutet Bezugszeichen 1 eine Beatmungsmaske, die auf Mund und Nase des Patienten aufgesetzt wird. Bezugszeichen 2 stellt ein patientennahes Adapterstück an der Beatmungsmaske dar für das Anbringen eines Sauerstoffsensors zur Durchführung des erfindungsgemäßen Verfahrens. Bezugszeichen 3 stellt weitere bekannte Bestandteile einer Beatmungsvorrichtung dar, die für die vorliegende Erfindung nicht von Relevanz sind und deren spezielle Ausgestaltung von der speziellen Beatmungsvorrichtung abhängt. Bezugszeichen 4 zeigt den Sauerstoffsensor, Bezugszeichen 5 die mit dem Sauerstoffsensor verbundene Auswerte- und Anzeigevorrichtung. In einer alternativen Ausführungsform zeigt Bezugszeichen 1 nicht eine Beatmungsmaske, sondern einen Patiententubus zur Beatmung eines Patienten. Ebenfalls können das Adapterstück 2 und die Beatmungsmaske bzw. Patiententubus 1 aus einem Teil bestehen.

Bei der Durchführung des erfindungsgemäßen Verfahrens wird die Beatmungsmaske 1 über Mund und Nase des Patienten angebracht. Beim Beatmen des Patienten wird über den schnellen Sauerstoffsensor 4 zunächst bei einem Atemzyklus der Sauerstoffpartialdruck (oder Sauerstoffgehalt) des Gasgemisches gemessen, das den Lungen des Patienten zugeführt wird, anschließend der Sauerstoffgehalt (bzw. Sauerstoffpartialdruck) in der Ausatemluft. Die Differenz der beiden Sauerstoffwerte entspricht im wesentlichen dem in der Ausatemluft vorhandenen Kohlendioxid, wenn unter den vorstehenden Annahmen eine einfache Umrechnung durchgeführt wird. Die entsprechenden Berechnungen werden in der Auswerteund Anzeigevorrichtung 5 durchgeführt und der Kohlendioxidwert wird direkt auf der Auswerte- und Anzeigevorrichtung 5 dargestellt. Das Verfahren wird kontinuierlich durchgeführt, so daß beim nächsten Atemzyklus erneut der Sauerstoffgehalt des in die Lungen des Patienten eintretenden sauerstoffhaltigen Gasgemisches bestimmt wird und entsprechend wieder der Sauerstoffgehalt der Ausatemluft bestimmt wird. Die Auswerte- und Anzeigevorrichtung bestimmt das Maximum des Kohlendioxidgehalts in der Ausatemluft und zeigt diesen Wert an. Es ist möglich, einen über mehrere Atemzyklen gemittelten Wert des maximalen Kohlendioxidgehalts anzugeben. Über eine Trendanzeige auf der Anzeigevorrichtung wird angezeigt, ob der Kohlendioxidgehalt im Laufe der Zeit ansteigt, abfällt oder konstant bleibt.

Bei guter Auflösung des Sauerstoffsensors sind die der Auswerteeinheit von dem Sauerstoffsensor zugeführten elektrischen Signale invers zu den Kurven der Figur 1. Die Auswerteeinheit invertiert die Daten und gibt dann entweder das komplette Kapnogramm aus oder den Maximalwert des Kohlendioxidgehalts und die Trendinformation.

## Patentansprüche

1. Verfahren zur atemzugsaufgelösten Bestimmung von Kohlendioxid in der Atemluft, bei dem über einen Sauerstoffsensor kontinuierlich der Sauerstoffpartialdruck in der Atemluft bestimmt wird und der Absolutwert der Differenz zwischen dem momentanen Sauerstoffpartialdruck in der Ausatemluft und dem Sauerstoffpartialdruck in der Einatemluft als Kohlendioxidgehalt in der Ausatemluft angegeben wird, **dadurch gekennzeichnet, daß** bei jedem Atemzyklus der Minimalwert des Sauerstoffpartialdrucks in der Ausatemluft bestimmt wird und der Absolutwert der Differenz des Minimalwerts des Sauerstoffpartialdrucks in der Ausatemluft und des Sauerstoffpartialdrucks in der Einatemluft als Maximalwert des Kohlendioxidgehalts in der Ausatemluft angezeigt wird.

2. Verfahren nach Anspruch 1, **dadurch gekennzeichnet, daß** der Maximalwert des Kohlendioxidgehalts in der Ausatemluft über mehrere Atemzyklen verglichen wird und die Änderung des Maximalwerts des Kohlendioxidgehalts in der Ausatemluft über mehrere Atemzyklen als Trendinformation angezeigt wird.

3. Verfahren nach einem der Ansprüche 1 oder 2, **dadurch gekennzeichnet, daß** der Sauerstoffsensor eine Ansprechzeit von nicht mehr als 500 Millisekunden aufweist.

4. Verfahren nach einem der Ansprüche 1 bis 3, **dadurch gekennzeichnet, daß** der Sauerstoffsensor ein elektrochemischer Sauerstoffsensor ist.

5. Beatmungsvorrichtung mit einem Adapter (2), einer Beatmungsmaske oder einem Patiententubus (1), einem Sauerstoffsensor (4) und einer Auswerte- und Anzeigevorrichtung (5), **dadurch gekennzeichnet, daß** die Auswerte- und Anzeigevorrichtung derart ausgestaltet ist, daß bei jedem Atemzyklus der Minimalwert des Sauerstoffpartialdrucks in der Ausatemluft bestimmt wird und der Absolutwert der Differenz des Minimalwerts des Sauerstoffpartialdrucks in der Ausatemluft und des Sauerstoffpartialdrucks in der Einatemluft als Maximalwert des Kohlendioxidgehalts in der Ausatemluft angezeigt wird.

6. Beatmungsvorrichtung nach Anspruch 5, **dadurch gekennzeichnet, daß** sie eine Beatmungsmaske (1) aufweist.

7. Beatmungsvorrichtung nach Anspruch 5, **dadurch gekennzeichnet, daß** sie einen Patiententubus (1) aufweist.

## Claims

1. Method for determining carbon dioxide in respiratory air per breath, in which method the oxygen partial pressure in the respiratory air is determined continuously via an oxygen sensor, and the absolute value of the difference between the instantaneous oxygen partial pressure in the exhaled air and the oxygen partial pressure in the inhaled air is indicated as the carbon dioxide content in the exhaled air **characterized in that** the minimum value of the oxygen partial pressure in the exhaled air is determined upon each respiratory cycle and the absolute value of the difference of the minimum value of the oxygen partial pressure in the exhaled air and the oxygen partial pressure in the inhaled air is indicated as maximum value of the carbon dioxide content in the exhaled air.

2. Method according to claim 1, **characterized in that** the maximum value of the carbon dioxide content in the exhaled air is compared over several respiratory cycles and that the change in the maximum value of the carbon dioxide content in the exhaled air is indicated over several respiratory cycles as trend information.

3. Method according to either claim 1 or 2, **characterized in that** the oxygen sensor has a response time of not more than 500 milliseconds.

4. Method according to any of claims 1 to 3, **characterized in that** the oxygen sensor is an electrochemical oxygen sensor.

5. Breathing device with an adapter (2), a breathing mask or a patient tubing (1), an oxygen sensor (4) and a plotting and display device (5), **characterized in that** the plotting and display device is adapted so that with each respiratory cycle the minimum value of the oxygen partial pressure in the exhaled air is determined and the absolute value of the difference between the minimum value of the oxygen partial pressure in the exhaled air and the oxygen partial pressure of the inhaled air is indicated as maximum value of he carbon dioxide content in the exhaled air.

6. Breathing device according to claim 5, **characterized in that** it has a breathing mask (1).

7. Breathing device according to claim 5, **characterized in that** it has a patient tubing (1).

## Revendications

1. Procédé de détermination, décomposée par respiration, de dioxyde de carbone dans l'air respiré, dans lequel, par l'intermédiaire d'un détecteur d'oxygène, la pression partielle d'oxygène est déterminée en continu dans l'air respiré et la valeur absolue de la différence entre la pression partielle d'oxygène momentanée dans l'air expiré et la pression partielle d'oxygène dans l'air inhalé est indiquée sous la forme de teneur en dioxyde de carbone dans l'air expiré, **caractérisé en ce que**, dans chaque cycle de respiration, la valeur minimale de la pression partielle d'oxygène est déterminée dans l'air expiré et la valeur absolue de la différence entre la valeur minimale de la pression partielle d'oxygène dans l'air expiré et la pression partielle d'oxygène dans l'air inhalé est indiquée comme valeur maximale de la teneur en dioxyde de carbone dans l'air expiré.

2. Procédé suivant la revendication 1, **caractérisé en ce que** la valeur maximale de la teneur en dioxyde de carbone dans l'air expiré est comparée sur plusieurs cycles de respiration et **en ce que** la modification de la valeur maximale de la teneur en dioxyde de carbone dans l'air expiré sur plusieurs cycles de respiration est indiquée comme une information de tendance.

3. Procédé suivant l'une des revendications 1 et 2, **caractérisé en ce que** le détecteur d'oxygène présente un temps de réponse qui ne dépasse pas 500 millisecondes.

4. Procédé suivant l'une des revendications 1 à 3, **caractérisé en ce que** le détecteur d'oxygène est un détecteur d'oxygène électrochimique.

5. Dispositif respiratoire comprenant un adaptateur (2), un masque respiratoire ou un tube pour patient (1), un détecteur d'oxygène (4) et un dispositif d'évaluation et d'affichage (5), **caractérisé en ce que** le dispositif d'évaluation et d'affichage est réalisé de telle façon que, à chaque cycle de respiration, la valeur minimale de la pression partielle d'oxygène dans l'air expiré est déterminée et la valeur absolue de la différence entre la valeur minimale de la pression partielle d'oxygène dans l'air expiré et la pression partielle d'oxygène dans l'air inhalé est indiquée comme valeur maximale de la teneur en dioxyde de carbone dans l'air expiré.

6. Dispositif respiratoire suivant la revendication 5, **caractérisé en ce qu'**il présente un masque respiratoire (1).

7. Dispositif respiratoire suivant la revendication 5, **caractérisé en ce qu'**il présente un tube pour patient (1).
